# EUROPEAN PATENT SPECIFICATION

(11) **EP 2 342 172 B1**
(45) Date of publication and mention of the grant of the patent: **08.08.2012**
(21) Application number: 09760015.9
(22) Date of filing: 02.10.2009
(51) Int. Cl.: C07C 45/67, C07C 49/323, C07F 5/04

(54) **BIFLUORENYLIDENE DERIVATIVES, THEIR PREPARATION AND USES THEREOF**
BIFLUORENYLIDENDERIVATE, IHRE HERSTELLUNG UND ANWENDUNGEN DAVON
DÉRIVÉS DE BIFLUORÉNYLIDÈNE, LEUR PRÉPARATION ET LEURS UTILISATIONS

(30) Priority: 02.10.2008 IT RM20080522
(43) Date of publication of application: 13.07.2011
(73) Proprietor: Università degli Studi di Roma "La Sapienza", 00185 Roma (IT)
(72) Inventor: MATTIELLO, Leonardo, 00161 Roma (IT); BAGALA' RAMPAZO, Liliana, I-00185 Romas RM (IT)
(74) Representative: Raimondi, Adriana
(86) International application number: PCT/IT2009/000448
(87) International publication number: WO 2010/038251

(56) References cited:
- JP-A- 11 054 284
- MINABE ET AL: "Formation of 2,2'-diacyl-9,9'-bifluorenylidene isomers from 2-acyl-9-bromofluorene and base-catalysed isomerisation of the formed alkenes" BULLETIN OF THE CHEMICAL SOCIETY OF JAPAN., vol. 79, no. 11, 2006, pages 1758-1765, XP002550684 CHEMICAL SOCIETY OF JAPAN, TOKYO.

## Description

### Field of the Invention

The present invention relates to bifluorenylidene derivatives, also defined with BFD in the following text, of the general formula (II), to the method of synthesis of said compounds, and to their use, in particular to their use as components of materials in the molecular electronics, spintronics and in the telecom fields.

### Prior Art

The aromatic compound bifluorenylidene, also BFD, of the formula (I) is known (CAS Reg. N° 746-47-4. 1; Japp., Francis, R. Journal of the Chemical Society Transactions (1880), 37, 83-90; Agranat, I. et al; J. Am. Chemical Society 1972, 94, 2889).

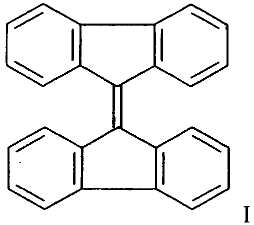

The Japanese Patent Application JP 11054284 relates to BFD compounds substituted in a number of ways (mono-, di-, tri-, and tetra-substituted) to be used as electroluminescent materials.

BFD and its derivatives are among those classes of organic molecules which can be used instead of inorganic species in molecular electronics, in particular for the arrangement and the manufacture of electroluminescent devices.

Tetra-derivatives of BFD have now been found which are highly symmetric and show chemical-physical features that are particularly interesting in terms of their use in the molecular electronics field. These derivatives are characterized by the fact that all their substituents are identical to each other.

### Summary of the Invention

Therefore object of the present invention are the BFD derivatives of the general formula (II): 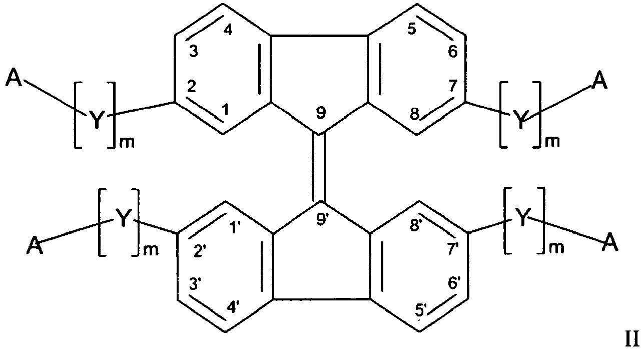 wherein:
Y = C=O and m is an integer ≥0, preferably m = 1, with the proviso that when m = 0, A = boranyl;
   - A = H, OH, alogen, amino group, thiol group, alkyl, oxyalkyl, alkenyl, alkynyl, aryl, boranyl.

In particular:
the alkyl group is R with R = linear, branched or cyclic aliphatic C₁-Cₙ, whith n being an integer >0, preferably C₁-C₂₀, preferably C₁-C₇, more preferably C₁-C₄;
the oxyalkyl group is an O-R group with R = linear, branched or cyclic aliphatic C₁-Cₙ, whith n being an integer >0, preferably C₁-C₂₀, preferably C₁-C₇, more preferably C₁-C₄;
the aryl group is an aromatic or susbstituted aromatic Ar group, possibly condensed, possibly containing heteroatoms, being the Ar substituted eventually with halogen groups and/or aliphatic chains R', wherein R' = R with R having the meanings mentioned above;
the amino group is a N(R₁, R₂), wherein R₁, R₂, whether identical or different from each other, are H, or have the meanings given to R or Ar;
the thiol group is an S-R" group with R" = H, or R" = R or Ar with R and Ar having the meanings mentioned above;
the boranyl is a B(OR₃, OR₄) group, wherein R₃ and R₄, whether identical or different from each other, are H, or have the meanings given to R or Ar; R₃ and R₄, may also be linked to each other to make a cycloalkyl, possibly branched.

In particular, A can be: hydrogen, chlorine, or an alkyl, aryl, hydroxyl, mercapto, amino, fluoroaryl, chloroaryl, nitroaryl, aminoaryl, alkoxyaryl, alkylaryl, N-alkylcarbazolyl, biphenylenyl, hydroxyaryl, mercaptoaryl, 2-alkyl fluorenone, alkyloxy, cyanoaryl, N(alkyl)₂, alkynyl, alkenyl, cyclopentadienonyl, furanyl, thiophenyl group, boranyl radical of the boric acid B(OH)₃ or of boric acid esters B(OR)₃ wherein R has the meaning mentioned above, boranyl radical of dioxaborolane and its derivatives, in particular 4,4,5,5-tetramethyl-1,3,2-dioxaborolane.

Another object of the invention are the radical-anions and radical-cations, in particular mono-, di-, and tri-anions, which correspond to the compounds of formula (II). It is understood that by radical-anion it is meant the chemical species obtained by addition of one or more electrons to the corresponding neutral species and, correspondingly, radical-cation the chemical species obtained by subtraction of one or more electrons from the corresponding neutral species.

Still another object of the invention are the method for manufacturing the compounds of formula (II) and the method for manufacturing the corresponding radical-anion and/or radical-cations, alone or in a mixture thereof.

Still another object of the invention are the compositions comprising the compounds of formula (II) and/or their corresponding anions and/or cations, alone or in a mixture thereof, to be used in the manufacture of electronic components.

A further object of the invention are the electronic devices which contain the compounds of formula (II) of the invention, and/or their corresponding radical anions and/or radical-cations, alone or in a mixture thereof.

Still a further object of the invention is the use of the BFD derivatives and of their corresponding radical anions and/or radical-cations, alone or in a mixture thereof, in the manufacture of electronic components.

Further objects will be apparent from the detailed description of the invention.

### Detailed Description of The Invention

The present invention relates to a particular class of BFD derivatives of formula (II), i.e., a class of highly symmetric compounds, being all the substituents A identical to each other, and symmetrically placed onto the BFD core in position 2,2',7,7'.

Particularly preferred are the derivatives wherein a carbonyl C=O group is directly bound to the core structure (BFD).

Another particularly preferred class of compounds is the one wherein A is a boranyl radical; the boranyl radical of the dioxaborolane being particularly preferred, more particularly the 4,4,5,5-tetramethyl-1,3,2-dioxaborolane-2-yl of formula (III): 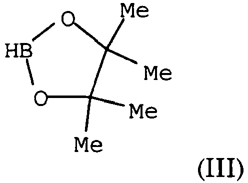 being preferred.

The boranyl radical directly bound to BFD (without C=O) may be considered as a reaction intermediate for the synthesis of useful compounds for the aforementioned aims, by applying the Suzuki synthesis (Miyaura, N. e Suzuki, A. Chem. Rev. 1995 95:2457-2483).

A method for the synthesis of the compounds of the invention comprises the following steps:
(i) synthesizing a di-substituted fluorene at positions 2 and 7 using fluorene as the starting compound and the compounds useful for its functionalization; useful synthetic ways to get to this reaction step usually are acylation reactions of Friedel Crafts using for example AlCl₃ as the catalyzer or equivalent catalysts; such reactions are known to the expert in the art and can be carried out starting from compounds which are easily found in the market or are easily synthesized. The synthetic reactions are described in the basic organic synthesis textbooks and many of the starting compounds for the syntheses herein described are commercially available;
(ii) adding an halogen imide to the reaction mixture containing the di-substituted fluorene synthesized in step (i), preferably N-bromosuccinimide and irradiating with UV light;
(iii) treating the compound obtained in step (ii) with alkaline or alkaline earth metals in an anhydrous environment, thus obtaining the final compound to be purified.

Preferably, the di-substituted fluorene of step (i) is purified by means of column chromatography, e.g., by using a dichloromethane-hexane 10% mixture as the eluent.

Preferably the irradiation is carried out in an aprotic environment, e.g., with carbon tetrachloride and a solar lamp at 300 watt for about 2 hours; the solution is then treated with water, which is subsequently separated from the organic phase, which is evaporated to yield the product to be treated in the subsequent step (iii).

Preferably step (iii) is carried out in a polar aprotic anhydrous solvent, e.g., anhydrous ethyl ether, in a nitrogen flow and under stirring. Then the solution is refluxed for the time sufficient to obtain the final product to be purified; the mixture is then cooled to room temperature, water is added slowly and from the organic phase the final product is recovered, purified by evaporation. Possibly, a further purification is carried out, e.g., by means of a column chromatography by using a dichloromethane-hexane 60% mixture as the eluent.

Within the scope of the present invention, with the term radical anion a chemical species is meant which is able to acquire an electron keeping its negative charge and radical feature. Analogously, a compound which has been deprived of an electron, carrying a positive charge, is called radical-cation. The radical-anion and radical-cation species can be obtained by a chemical or electrochemical route ["Organic Electrochemistry", 4a Ed., Henning Lund & Ole Hammerich Eds., Marcel Dekker Inc, New York (2001), and *"*Electrochemical methods", 2a Ed., A.J. Bard, L.R. Faulkner, Wiley, New York (2001)].

A radical-anion shows a reversible behaviour when, upon inversion of the applied potential sign to an appropriate value, it yields back the starting compound (see the examples reported in the technical references cited above). The same is true with the radical-cation.

The radical-anions of the compounds of the invention are preferably obtained by a chemical or electrochemical route, by addition of an electron to the corresponding neutral compound; particularly preferred is the electrochemical route for its selectivity and ease of execution.

To obtain di-anion and tri-anion radicals, which can be paramagnetic species, it is enough to work at more negative potentials with respect to those used to obtain the respective mono- or di- anions, as indicated in the experimental protocols.

The general electrochemical method to obtain the radical-anions is described in:
- "Organic Electrochemistry", 4a Ed., Henning Lund & Ole Hammerich Eds., Marcel Dekker Inc, New York (2001).
- "Electrochemical methods", 2a Ed., A.J. Bard, L.R. Faulkner, Wiley, New York (2001).

The optimal conditions for obtaining the desired compounds are accessible to the expert in the field.

The compounds of the invention, thanks to the presence of the C=O group bound to BFD, yield more easily radical-anions with respect to the correspondent compounds wherein the C=O is not present.

The molecular structure of the compounds of the invention shows chemical-physical features mainly connected to the molecular symmetry and are particularly interesting for the use in the molecular electronics, in the spintronics and in the telecom fields.

The tetra-substituted BFD derivatives according to the invention are preferred with respect to the correspondent di-substituted ones with the same substituents due to the favourable conjugation situation which characterizes them.

The compounds of the invention can be applied as coatings or thin films onto appropriate supports (either metallic or non-metallic) by means of known techniques (chemical, chemical-physical, physical) known to the expert in the field. The electronic devices which incorporate them and/or onto which they are layered carry at least one active layer which comprises at least one compound of the invention, preferably mixtures, possibly comprising the radical-anions and/or cations, applied onto said supports.

The compounds can be applied onto the supports of electronic devices by means of sublimation or by deposition techniques in vapour phase (e.g., OVPD - Organic Vapour Phase Deposition) or by spin-coating or by printing techniques such as offset or ink-jet (all of which are techniques known to the expert in the field).

With the general term "molecular electronics" it is meant the technical field according to which molecular organic species can be used for electronic applications, being electroluminescence, photoluminescence and both electronic and holes conductivity comprised within the meaning of this term.

With the general term "spintronic" it is meant the technical field according to which molecular organic species can be used exploiting the spin properties of the electrons which are present within the molecule. In other words, in the electronic devices of the molecular electronics the information is transmitted and stocked by the electric flow in the form of subatomic particles negatively charged, called electrons, or positively charged, called holes. The binary code zero and one in a computer are represented by the presence or absence of electrons in a semi-conductor or in a different material. In molecular spintronic, the information is stocked and transmitted using a different electron property i.e., their spin.

The use of the compounds herein claimed allows manufacture of more efficient nano-devices for the applications cited above.

The compounds derived from BFD which are the object of the present invention can be used as materials which transfer negative charges (electrons) or positive charges (holes) or as guest materials in electronic devices. In addition, said compounds can advantageously be used in electronic devices (e.g., OLEDS), i.e., devices for light-emitting diodes based on organic compounds, in particular emitting in the visible, in the blue and green-blue and emitting white light (nowadays used also in television screens), in the OFETS, i.e., field-effect transistors based on organic compounds, integrated circuits based on organic compounds, solar cells based on organic compounds, light-emitting transistors based on organic compounds, light-emitting electrochemical cells, organic photoreceptors and organic laser-diodes, electrochromic materials, organic spin valves, gaseous H₂ sensors.

The compounds herein claimed can further be used as active means in electroluminescent devices (with the inclusion of lasers) or photovoltaic devices and as materials to transport charges in electroluminescent devices, transistors, photovoltaic devices, in telecom devices, i.e., for the manufacture, transmission and detection of certain electromagnetic frequencies, and in general for the industrial production of metamaterials useful for the above mentioned applications. The compounds herein described can be used in combination with others as additional species in carriying out the above mentioned applications.

The compounds herein described can be used as additional species in carriying out the above mentioned applications.

In particular, the compounds mentioned above can be used as components of OLEDS, organic semi-conductors, field-effect transistors (OFETS), molecular rectifiers, organic molecules for laser applications, organic photovoltaic devices, organic spin valves, solar cells, electrochromic and thermochromic materials, in general for electronic components and devices on a molecular scale, components and devices for gaseous H₂ sensors, components and devices for the manufacture, transmission and detection of electromagnetic frequencies also in the field of far infra-red, components and devices for spintronics, for chemical sensors, and for the industrial manufacture of metamaterials in general, useful for the above mentioned applications.

It is known that the standard potential, E°, of an organic molecule shifts itself toward more positive values with respect to a reference molecule when its properties as an electron-acceptor are improved with respect to the reference molecule.

With reference to the compounds, derivatives and salts thereof, according to the present invention and to the corresponding radical-anions, the standard potential E° gets shifted toward more positive values by the quantity ΔE°.

By way of example, the increase ΔE° toward more positive potentials with respect to the corresponding compounded values not containing the functional group C=O carries the advantage of an energetic saving using the molecules of the invention. The compounds of the invention and their corresponding radical-anions, thanks to the presence of a plurality of conjugated C=O groups can advantageously be employed in the field of electroluminescence in general, particularly for light-emitting diodes (OLEDS), more in particular blue-light OLEDS, as components of molecular switchings, for non-linear optics, in molecular-based computer system, in field-effect transistors (FET), in negative differential resistance semi-conductors (NDR). Exactly because of the presence of a number of conjugated C=O groups, the compounds of the invention allow an easy transfer of a greater number of electrons with respect to similar compounds, thus allowing the manufacture of anionic species which can be used as molecular magnets.

The following examples are given by way of example of the invention and are not to be considered as limiting its scope.

### Example 1: Synthesis of 2,7-dibenzoylfluorene.

293 mg of anhydrous, finely pulverized AlCl₃ (P.F. = 133.30; 2.2 mmol) are added to 281 mg of benzoyl chloride (P.F. = 140.57; 2 mmol) in 10 ml of CS₂ at 0°C (water-ice bath). A solution containing 166 mg of fluorene (P.F. = 166.22; 1 mmol) in 5 ml of CS₂ is added dropwise under stirring, during a time period of half an hour, and is let to cool at room temperature. A reflux is created and the stirring is maintained for further 2 hours. Water and ice are then added, then diluted HCl, and the organic phase is separated. The organic extracts are pooled and treated with a saturated solution of sodium carbonate, washed with water and dried on anhydrous sodium sulphate. A column chromatography follows, eluting with a 10% dichloromethane-hexane mixture (yield ∼75%).
¹H-NMR (CDCl₃, 200 MHz, δ vs SiMe₄): 8.20 - 7.45 (16 H, mc, ArH); 3.98 (2H, s, CH₂ fluorene).
¹³C-NMR (CDCl₃, 50 MHz, δ vs SiMe₄): 36.94 (CH₂ fluorene); 120.39; 126.88; 128.29; 128.41; 129.63; 129.95; 130.13; 132.33; 133.63; 136.94; 137.91; 144.20; 144.54 (all quaternary aromatic C and aromatic CH); 196.49 (CO).

### Example 2: Synthesis of 2,7-dibenzoyl-9,9'-dibromofluorene.

Anhydrous carbon tetrachloride (10 ml), 100 mg (0.267 mmol, P.F. = 347) of 2,7-dibenzoylfluorene and 96 mg of N-bromosuccinimide (0.534 mmol, P.F. = 178) were stirred for 2 hours and irradiated by means of a solar lamp (300 W). The solution is treated with water and the solvent is evaporated with the aid of a rotavapor (yield ∼ 95%).
¹H-NMR (CDCl₃, 200 MHz, δ vs SiMe₄): 8.94 - 6.52 (16H, mc, ArH)
¹³C-NMR (CDCl₃, 50 MHz, δ vs SiMe₄): 67.51 (C-9 fluorene); 120.19; 128.33; 129.00; 130.02; 132.50; 137.08; 141.56 (all quaternary aromatic C and aromatic CH); 194.93 (CO).

### Example 3: Synthesis of 2,2',7,7'-tetrabenzoyl-9,9-bifluorenylidene TBBFD)

A suspension of 23 mg (0.94 mmol) of magnesium in pellets in 5 ml of anhydrous ethyl ether is added dropwise to a solution of 100 mg of 2,7-dibenzoyl-9,9'-dibromofluorene (0.188 mmol) in 10 ml of anhydrous ethyl ether (in a nitrogen flow and under stirring). The solution is then refluxed for 12 hours and is cooled to room temperature, and subsequently water is slowly added. The liquid phase is decanted from the salts which are washed with exane and later discarded. The pooled organic phases are washed with water, with a saturated aqueous solution of sodium hydrogen carbonate and with a saturated aqueous solution of sodium chloride. The solution is finally dried with anhydrous sodium sulphate and the solvent is evaporated with a rotavapor. A column chromatography follows using a mixture of dichloromethane-hexane 60% (yield - 25%).
¹H-NMR (CDCl₃, 200 MHz, δ vs SiMe₄): 8.70 - 7.36 (mc, ArH)
¹³C-NMR (CDCl₃, 50 MHz, δ vs SiMe₄): 120.74; 129.70; 132.19; 132.35; 137.36; 137.51; 138.92; 140.50; 143.76 (all quaternary aromatic C and aromatic CH); 195.72 (CO).

### Example 4: Synthesis of bifluorenylidene (BFD)

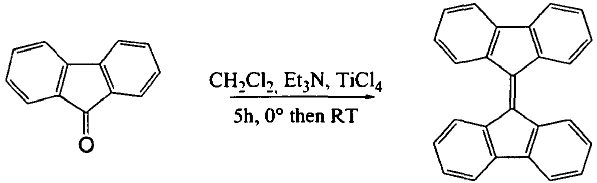

10 ml of anhydrous trichloromethane and 4.16 ml of triethylamine (30 nmol) are mixed under N₂ atmosphere at 0°C. 20 ml of a 1M solution of titanium tetrachloride in dichloromethane (20 mmol) are added under nitrogen at 0°C. The reaction is stirred for 1h at 0°C and to this reaction mixture 1.800 g of fluorenone are added (10 mmol) and the stirring is continued for further 5 hours at 0-25°C. The reaction mixture is then quenched with a saturated solution of NH₄Cl (20 ml). The organic layer is separated and the aqueous layer is extracted with CH₂Cl₃ (3 x 30 ml). The pooled organic extracts are washed with brine (20 ml) and dried on anhydrous Na₂SO₄. The solvent is removed and the residue is chromatographed on a silica gel column.

### Example 5: Synthesis of 2,2',7,7'-tetrabromobifluorenylidene (TBrBFD)

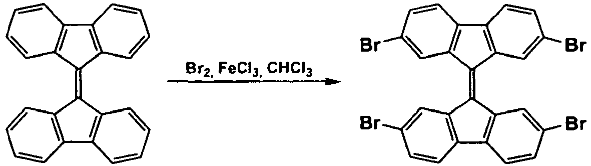

50 mg (0.3 mmol) of FeCl₃ and 12 ml (24 mmol) of bromine are added to 1.00 g (3.0 mmol) of bifluorenylidene dissolved in 25 ml of chloroform at 0°C. The solution is refluxed for three hours and stirred overnight at RT. Water is then added and the mixture is washed with some saturated solution of sodium thiosulphate until the red color disappears. The mixture is then extracted several times with CH₂Cl₂ and dried on anhydrous Na₂SO₄.

### Example 6: General synthetic process for aryl boronic acids

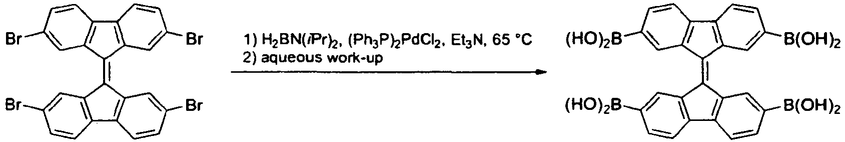

In a 100 ml round-bottom flask equipped with two necks, triethylamine (25 mmol), aryl bromide (e.g., TBrBFD) (5 mmol) and THF (3 ml) are placed together. The septum is substituted with a reflux condenser while palladium is added (0.5 mmol, 5 mol%). Finally, to the reaction mixture di-isopropylaminoborane is added with a syringe (15 ml, 1 M solution in THF, 15 mmmol). The reaction mixture is then heated under reflux at 65°C. After a 19 h reflux the solution is left to cool to 25°C, and methanol is slowly added (8 ml). The solvent is evaporated under vacuum (25 torr), and the residue is dissolved with sodium hydroxide (3 M, 8 ml). The aqueous layer is washed with hexane (3 x 10 ml) and subsequently acidified with 3 M HCl (pH 1). In many cases boronic acid precipitates out of the solution. The mixture is extracted with diethyl ether (4 x 15 ml). The organic portions are reunited, dried with anhydrous MgSO₄ and filtered. The solvent is evaporated under vacuum to yield the desired arylboronic acid (e.g., the boronic derivative of BFD).

### Example 7: General cross-Coupling reaction prowess of ferrocenylimine cyclopalladate of arylboronic reagents with terminal alkynes.

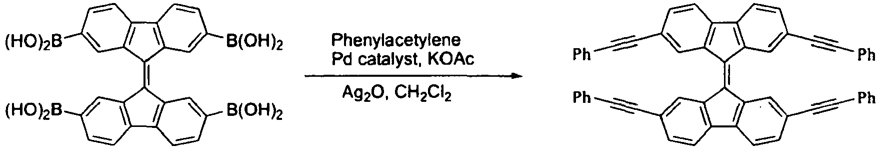

A mixture of the aryl boronic reagent (e.g., the boronic derivative of BFD) (0,5 mmol), alkyne (0.6 mmol), the catalyzer ferrocenylimine cyclopalladate (1 mol-%), Ag₂O (0.5 mmol), KOAc (0.75 mmol) and CH₂Cl₂ (3 ml) are stirred at room temperature under a nitrogen atmosphere, and the reaction is followed by TLC or GC. After the completion of the reaction, the mixture is filtered and the solvent is removed under reduced pressure. The residue is purified by column chromatography (hexane or hexane/ethyl acetate) to yield the desired product (e.g., the alkyne BFD derivative).

### Example 8: General synthetic process for the phenyl derivative of BFD.

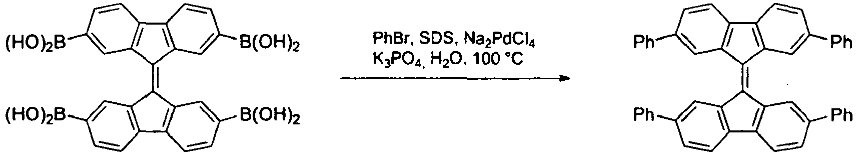

Aryl boronic acid (e.g., the boronic derivative of BFD) (1.2 mmol) is added under stirring to a solution of Na₂PdCl₄ (2.5 mol%), K₃PO₄ (1.5 mmol) and SDS (sodium dodecyl sulphate) (0.5 mmol) in water followed by aryl bromide (1 mmol) at room temperature under air. The reaction mixture is heated to 100°C (oil bath) for 5 min. The extraction with ethyl acetate and the purification (re-crystallization/chromatography) yield a pure product (e.g., the phenyl derivative of BFD).

### Example 9: General procedure of Suzuki cross-coupling of acyl halides and boronic acids.

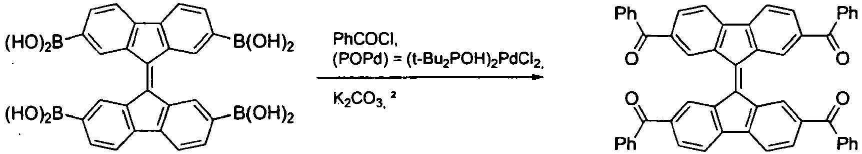

Phenyl boronic acid (181 mg, 1.4 mmol), benzoyl chloride (150 mg, 1.05 mmol), POPd (2.5 mol%) and K₂CO₃ (241 mg, 1.7 mmol) are dissolved in 1.75 ml of toluene: 1,4-dioxane (2:1, v/v). The reaction mixture is heated to 80°C for 1 hour, quenched with 1 ml of water and extracted with methylene chloride. The pooled organic layers are dried on anhydrous MgSO4 and concentrated *in vacuo.* The crude product is purified by flash chromatography.
Examples 7 and 8 are given to show that the boronic derivatives of BDF can be used as an intermediate to obtain compounds which are out of the invention.

### Example 10: Synthesis of radical-anions

The radical-anions of the compounds of the invention are obtained preferably by a chemical or an electrochemical route by addition of an electron to the corresponding neutral compound; particularly preferred is the electrochemical route due to its selectivity and ease of execution.

To obtain di-radical di-anions, where possible, which may also be paramagnetic species, it is sufficient to operate at more negative potentials with respect to those used for radical anions, as indicated in the experimental conditions.

The electrochemical model to obtain radical-anions in general is described in :
- *"*Organic Electrochemistry", 4a Ed., Henning Lund & Ole Hammerich Eds., Marcel Dekker Inc, New York (2001).
- *"*Electrochemical methods", 2a Ed., A.J. Bard, L.R. Faulkner, Wiley, New York (2001).

Such a method is carried out by using an electrochemical cell comprising two sections: an anodic and a cathodic one; in the cathodic one a working electrode and a reference calomel electrode are placed. An aprotic solvent or mixtures of: typically N,N-dimethyl formamide, acetonitrile, tetrahydrofurane, N- methylpyrrolidone, dimethyl sulphoxide, preferably N,N-dimethylformamide, acetonitrile and, particularly preferred N,N-dimethylformamide, are made anhydrous according to the current procedures, and to them a supporting electrolyte is added, typically tetraethylammonium perchlorate, tetrabutylammonium tetrafluoroborate, litium perchlorate, particularly preferred tetraethylammonium perchlorate, also made anhydrous, so that a concentration ranging in between 1 M and 0.01 M, preferably 0.2 M and 0.05 M, particularly preferred about 0.1 M is obtained.

The electrolyte solution thus obtained is place in the cathodic section which is separated from the anodic one by a portion of the same electrolytic solution conveniently gelified and in which the anode is placed (Pt gauze).

The selected compound is added to the electrolytic solution in the cathodic section of a divided cell, under nitrogen flow, so that a concentration ranging in between 0.01 M and 1 mM, preferably ranging in between 0.01 M and 0.5 mM and particularly preferred 1 mM is obtained. In the cathodic section of the cell a reticulated vitreous carbon (RVC) electrode is placed as the cathode and a calomel electrode (SCE) as the reference electrode. In the anodic section of the cell, separated from the cathodic section by means of a gelified electrolytic solution, an electrode, preferably a platinum gauze, is placed as the anode. Other electrodic materials useful in the manufacture of working electrodes are: mercury, lead, silver, composite Ti-containing materials, carbon-containing conducting materials, chemically modified electrodes, particularly preferred is vitreous carbon for the following reasons: wide applicable electrochemical window, low price, absence of toxicity and ease of use.

The supporting electrolytes which can be used are the ones preferably containing: perchlorate anions, tetrafluoroborate anions, hexafluorophosphate anions, lithium cations, sodium cations, tetraalkylammonium cations and mixtures thereof; particularly preferred are perchlorate anions and tetra-ethylammonium cations.

An appropriate potential is applied in between the electrodes so that the desired radical-anion is obtained, usually a voltage of about 0.2 V more negative than the standard potential E° of the compound to be treated (vs SCE).

The working temperatures can be comprised in between -20°C and + 50°C; room temperature is particularly preferred.

An example of radical-anions (mono-, di-, and tri-anions) is given in the experimental section and their relative E° are expressed in the following Table 1:

**Table 1**

| Starting compound | E°₁ (V vs SCE*) | E°₂ (V vs SCE*) | E^{C}ₚ₃ (V vs SCE*) |
|---|---|---|---|
| TBBFD | -0.49 | -0.80 | -1.47 |

| | | | |
|---|---|---|---|
| *Saturated Calomel Electrode | | | |

## Claims

1. Bifluorenylidene derivatives and corresponding radical anions and cations of the following general formula (II): 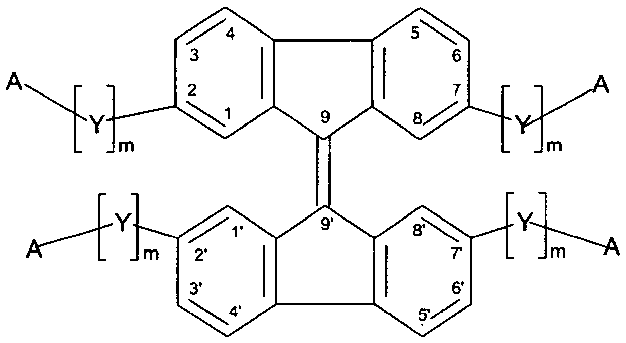 wherein:
Y = C = O and m is a positive integer ≥0, preferably m=1, with the proviso that when m=0, A=boranyl;
A = H, OH, halogen, amino group, thiol group, alkyl, oxyalkyl, alkenyl, alkynyl, aryl, boranyl.

2. Bifluorenylidene derivatives and corresponding radical-anions and radical-cations according to claim 1, wherein:
the alkyl group is R with R = linear, branched or cyclic aliphatic C₁-Cₙ, with n being an integer >0, preferably C₁-C₂₀, preferably C₁-C₇, more preferably C₁-C₄;
the oxyalkyl group is an O-R group with R = linear, branched or cyclic aliphatic C₁-Cₙ, with n being an integer >0, preferably C₁-C₂₀, preferably C₁-C₇, more preferably C₁-C₄;
the aryl group is an aromatic or susbsituted aromatic Ar group, possibly condensed, possibly containing eteroatoms, being the Ar substituted possibly with alogen groups and/or aliphatic chains R', wherein R' = R with R having the meaning mentioned above;
the amino group is a N(R₁, R₂) group, wherein R₁, R₂, whether identical or different from each other, are H, or have the meanings given to R or Ar;
the thiol group is an S-R" group with R" = H, or R" = R or Ar with R and Ar having the meanings mentioned above;
the boranyl is a B(OR₃, OR₄) group, wherein R₃ and R₄" whether identical or different from each other, are H, or have the meanings given to R or Ar; eventually being R₃ and R₄ also linked to each other to form a cycloalkyl, possibly a branched cycloalkyl.

3. Bifluorenylidene derivatives and corresponding radical-anions and radical-cations according to claims 1-2, wherein A is selected among: hydrogen, chlorine, alkyl, aryl, hydroxy, mercapto, amino, fluoroaryl, chloroaryl, nitroaryl, aminoaryl, alkoxyaryl, alkylaryl, N-alkylcarbazolyl, biphenylenyl, hydroxyaryl, mercaptoaryl, fluorenone-2-yl, alkyloxy, cyanoaryl, N(alkyl)z, alkynyl, alkenyl, cyclo-pentadienonyl, furanyl, thiophenyl, boranyl radical of boric acid B(OH)₃ or of boric acid B(OR)₃ esters wherein R has the meaning mentioned above, boranyl radical of dioxaborolan and its derivatives, in particular 4,4,5,5-tetramethyl-1,3,2-dioxaborolan.

4. Method for synthesizing the derivatives according to claims 1-3, comprising the following steps:
(i) synthesizing a di-substituted fluorene in position 2 and 7;
(ii) adding an halogen imide to the reaction mixture containing the di-substituted fluorene synthesized in step (i), preferably N-bromosuccinimide and irradiating with UV light;
(iii) treating the compound obtained in step (ii) with alkaline or alkaline earth metals in an anhydrous environment.

5. Electrochemical method for synthesizing the radical anions of the derivatives according to claims 1-3, **characterized in that** said derivatives to be transformed into radical anions are added at a concentration between 0,01M and 1 mM, preferably between 0.01 M and 0.5 mM, more preferably approximately 1 mM, to an anhydrous aprotic solvent containing a supporting electrolyte, also anhydrous, in order to obtain a concentration of the latter ranging between 1 M and 0.01 M, preferably 0.2 M and 0.05 M, more preferably about 0.1 M; being the mixture then placed in an electrolytic cell and a voltage being applied between the electrodes in order to obtain the required radical anion.

6. Electronic devices and components, selected among: OLEDS, organic semi-conductors, field-effect transistors (OFETS), molecular rectifiers, lasers; organic photovoltaic devices; organic spin valves; solar cells; electrochromic and thermochromic materials for electronic components and devices on a molecular scale; components and devices for gaseous H₂ sensors; components and devices for the production, transmission and detection of electromagnetic frequencies also in the far infra-red field; components and devices for spintronics, for chemical sensors and in general metamaterials useful for the above mentioned applications, comprising at least one of the derivatives and corresponding radical-anions and cations according to claims 1-3, and mixtures thereof.

7. Use of the derivatives and of the corresponding radical-anions and cations according to claims 1-3, alone or in a mixture thereof, as negative charge (electrons) or positive charge (holes) transfer materials or as guest materials in electronic devices.

8. Use of the derivatives and of the corresponding radical-anions and cations according to claim 7, wherein said electronic devices are selected among: OLEDS, i.e. devices for light-emitting diodes, in particular emitting in the visible, in the blue and the green-blue and emitting white light, in particular for television screens; OFETS, i.e., field-effect transistors based on organic compounds; integrated circuits based on organic compounds; solar cells based on organic compounds; light-emitting transistors based on organic compounds; light-emitting electrochemical cells; organic photoreceptors and organic laser-diodes; electrochromic materials, organic spin valves, gaseous H₂ sensors.

9. Use of the derivatives and corresponding radical-anions and cations according to claims 1-3, alone or in a mixture, as active means in photovoltaic devices and electroluminescence devices, including lasers; and as charge transfer materials in electroluminescence devices; transistors; telecom devices, i.e. for the production, transmission and detection of electromagnetic frequencies and in general for the industrial production of metamaterials useful for the applications mentioned above.

## Patentansprüche

1. Bifluorenyliden-Derivate und entsprechende Radikalanionen- und -kationen der folgenden allgemeinen Formel (II): 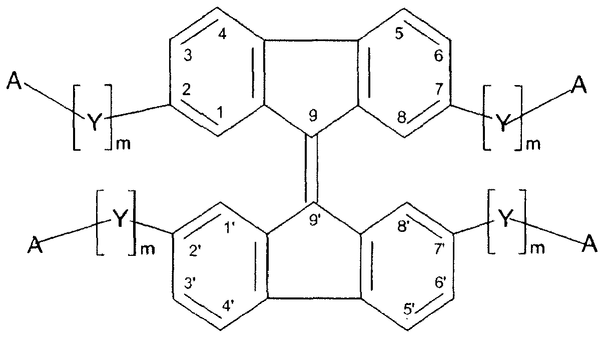 wobei:
Y = C = O ist und m eine positive ganze Zahl ≥ 0 ist, vorzugsweise m=1, mit dem Vorbehalt, dass, wenn m=0, A=Boranyl ist; und
A = H, OH, Halogen, Aminogruppe, Thiolgruppe, Alkyl, Oxyalkyl, Alkenyl, Alkynyl, Aryl, Boranyl ist.

2. Bifluorenyliden Derivate und entsprechende Radikalanionen- und -kationen gemäß Anspruch 1, wobei:
die Alkylgruppe R ist mit R = linearem, verzweigtem oder zyklisch aliphatischem C₁-Cₙ, mit n als ganze Zahl > 0, vorzugsweise C₁-C₂₀, vorzugsweise C₁-C₇, noch bevorzugter C₁-C₄:
die Oxylakylgruppe eine O-R Gruppe ist mit R = linearem, verzweigtem oder zyklisch aliphatischem C₁-Cₙ mit n als ganze Zahl > 0, vorzugsweise C₁-C₂₀, vorzugsweise C₁-C₇, noch bevorzugter C₁-C₄;
die Arylgruppe eine aromatische oder substituierte aromatische Ar Gruppe ist, möglicherweise kondensiert, möglicherweise Heteroatome enthaltend, wobei Ar möglicherweise mit Halogengruppen und/oder aliphatischen Ketten R' ersetzbar ist, wobei R'= R ist, wobei R die oben erwähnte Bedeutung hat;
die Aminogruppe eine N(R₁, R₂) Gruppe ist, wobei R₁, R₂, unabhängig davon ob sie identisch oder unterschiedlich zueinander sind, H sind, oder die R oder Ar die gegebenen Bedeutungen haben;
die Thiogruppe eine S-R" Gruppe ist mit R" = H, oder R" = R oder Ar, wobei R und Ar die oben erwähnten Bedeutungen haben; und
das Boranyl eine B(OR₃, Or₄) Gruppe ist, wobei R₃ und R₄, unabhängig davon ob sie identisch oder unterschiedlich zueinander sind, H sind, oder die R oder Ar die gegebenen Bedeutungen haben; wobei eventuell R₃ und R₄ auch miteinander verbunden sind um ein Cycloalkyl zu bilden, möglicherweise ein verzweigtes Cycloalkyl.

3. Bifluorenyliden Derivate und entsprechende Radikalanionen- und -kationen gemäß den Ansprüchen 1-2, wobei A ausgewählt wird aus: Wasserstoff, Chlor, Alkyl, Aryl, Hydroxy, Mercapto, Amino, Fluoraryl, Chloraryl, Nitroaryl, Aminoaryl, Alkoxyaryl, Alkylaryl, N-alkylcarbazolyl, Biphenylenyl, Hydroxyaryl, Mercaptoaryl, Fluorenon-2-yl-, Alkyloxy, Cyanoaryl, N(Alkyl)₂, Alkynyl, Alkenyl, Cyclo-pentadienonyl, Furanyl, Thiophenyl, Boranylradikale von Borsäure B(OH)₃ oder von Borsäure B(OR)₃ Estern, wobei R die oben erwähnte Bedeutung hat, Boranylradikale von Dioxaborolan und seinen Derivaten, insbesondere 4,4,5,5-Tetramethyl-1,3,2-Dioxaborolan.

4. Verfahren zur Herstellung der Derivate gemäß den Ansprüchen 1-3, die folgenden Schritte aufweisend:
(i) Synthetisieren eines di-substituierten Fluorens in Position 2 und 7;
(ii) Zugabe eines Halogen-Imids zur Reaktionsmischung, beinhaltend die in Schritt (i) di-substituierten Fluorene, vorzugsweise mit UV-Licht bestrahlte N-Bromsuccinimide; und
(iii) Behandeln der in Schritt (ii) erhaltenen Verbindung mit Alkali- oder Erdalkalimetallen in einer wasserfreien Umgebung.

5. Elektrochemisches Verfahren für die Synthese der Radikalanionen der Derivate gemäß den Ansprüchen 1-3, **dadurch gekennzeichnet, dass** diese in Radikalanionen umzuwandelnde Derivate in einer Konzentration zwischen 0,01 M und 1 mM, vorzugsweise zwischen 0.01 M und 0.5 mM, noch bevorzugter ungefähr 1 mM, zu einem wasserfreien aprotischen, ein ebenfalls einen wasserfreien Grundelektrolyt enthaltendes, Lösungsmittel, hinzugegeben werden, um eine Konzentration der letzteren im Bereich zwischen 1 M und 0.01 M, vorzugsweise 0.2 M und 0.05 M, noch bevorzugter ungefähr 0.1 M, zu erhalten, wobei die Mischung dann in einer elektrolytischen Zelle angeordnet wird und eine Spannung zwischen den Elektroden angelegt wird, um das erforderliche Radikalanion zu erhalten.

6. Elektronische Geräte und Komponenten, ausgewählt unter: OLEDs, organischen Halbleitern, Feldeffekt-Transistoren (OFETs), molekularen Gleichrichtern, Lasern; organischen Photovoltaik-Geräten; organischen Spin-Ventilen; Solarzellen; elektrochromen und thermochromen Materialien für elektronische Komponenten und Geräte einer molekularen Größenordnung; Komponenten und Geräte für H₂-Gassensoren; Komponenten und Geräte für die Erzeugung, Übertragung und Erfassung von elektromagnetischen Frequenzen auch im fernen Infrarot-Bereich; Komponenten und Geräte für die Spintronik, für chemische Sensoren und im Allgemeinen Metamaterialien, welche für die oben erwähnten Anwendungen nützlich sind, umfassend mindestens eines der Derivate und den entsprechenden Radikalanionen- und -kationen gemäß den Ansprüchen 1-3, und Mischungen davon.

7. Verwendung der Derivate und der entsprechenden Radikalanionen- und -kationen gemäß der Ansprüche 1-3, alleine oder in einer Mischung davon, als negativ geladene (Elektronen) oder positiv geladene (Löcher) Transfermaterialien oder als Gastmaterialien in elektronischen Geräten.

8. Verwendung der Derivate und der entsprechenden Radikalanionen- und -kationen gemäß Anspruch 7, wobei diese elektronischen Geräte ausgewählt werden aus: OLEDs, d.h. Geräte für Leuchtdioden, insbesondere im sichtbaren emittierende, im blauen und grün-blauen und weißen Licht emittierende, insbesondere für Fernsehbildschirme; OFETs, d.h. Feld-Effekt-Transistoren basierend auf organischen Verbindungen; integrierten Schaltungen basierend auf organischen Verbindungen; Solarzellen basierend auf organischen Verbindungen; lichtemittierenden Transistoren basierend auf organischen Verbindungen, lichtemittierenden elektrochemischen Zellen; organischen Photorezeptoren und organischen Laserdioden; elektrochromen Materialien, organischen Spin-Ventilen, H₂-Gassensoren.

9. Verwendung der Derivate und der entsprechenden Radikalanionen- und -kationen gemäß der Ansprüche 1-3, alleine oder in einer Mischung, als aktive Mittel in Photovoltaikgeräten und Elektrolumineszenzvorrichtungen, einschließlich Lasern; und als Ladungstransfer-Materialien in Elektrolumineszenzvorrichtungen; Transistoren; Telekom-Geräten, d.h., für die Erzeugung, Übermittlung und Erfassung von elektromagnetischen Frequenzen und generell für die industrielle Herstellung von Metamaterialien nützlich für die oben genannten Anwendungen.

## Revendications

1. Dérivés de bifluorénylidène et anions et cations radiculaires correspondants de la formule générale suivante (II): 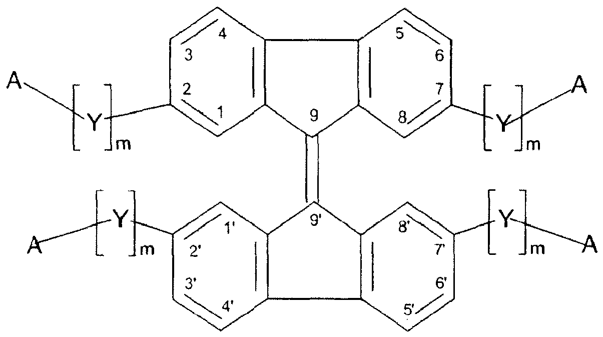 dans laquelle:
Y = C = O et m est un nombre entier positif ≥ 0, de préférence m = 1, avec la condition que lorsque m = 0, A = boranyle; et
A = H, OH, un halogène, groupe amino, groupe thiol, alkyle, oxyalkyle, alcényle, alcynyle, aryle, boranyle.

2. Dérivés de bifluorénylidène et anions et cations radiculaires correspondants selon la revendication 1, dans laquelle :
le groupe alkyle est un groupe R avec R = C₁-Cₙ linéaire, ramifié ou cyclique aliphatique avec n étant un nombre entier > 0, de préférence C₁ -C₂₀, de préférence C₁-C₇, plus préférentiellement C₁-C₄;
le groupe oxyalkyle est un groupe OR avec R = C₁-Cₙ linéaire, ramifié ou cyclique aliphatique avec n étant un nombre entier > 0, de préférence C₁-C₂₀, de préférence C₁-C₇, plus préférentiellement C₁-C₄;
le groupe aryle est un groupe Ar aromatique ou aromatique substitué, éventuellement condensé, contenant éventuellement des hétéroatomes, où Ar peut être substitué éventuellement avec des groupes halogènes et/ou des chaînes aliphatiques R', où R' = R avec R ayant la signification mentionnée ci-dessus;
le groupe amino est un groupe N(R₁, R₂), dans lequel R₁, R₂ sont soit identiques ou différents les uns aux autres, sont H, ou ont les significations données à R ou à Ar;
le groupe thiol est un groupe SR" avec R" = H, ou R" = R ou Ar avec R et Ar ayant les significations mentionnées ci-dessus;
le boranyle est un groupe B(OR₃, OR₄), dans lequel R₃ et R₄ sont soit identiques ou différents les uns aux autres, sont H, ou ont les significations données à R ou
Ar; éventuellement R₃ et R₄ étant également liés l'un à l'autre pour former un groupe cycloalkyle, éventuellement un cycloalkyle ramifié.

3. Dérivés de bifluorénylidène et anions et cations radiculaires correspondants selon l'une quelconque des revendications 1-2, dans lesquels A est choisi parmi:
hydrogène, chlore, alkyle, aryle, hydroxy, mercapto, amino, fluoroaryle, chloroaryle, nitroaryle, aminoaryle, alcoxyaryle, alkylaryle, N-alkylcarbazolyle, biphénylényle, hydroxyaryle, mercaptoaryle, fluorénone-2-yle, alkyloxy, cyanoaryle, N(alkyle)₂, alcynyle, alcényle, cyclopentadienonyle, furanyle, thiophényle, boranyle radiculaire de l'acide borique B(OH)₃ ou un ester de l'acide borique B(OR)₃, où R a la signification mentionnée ci-dessus, boranyle radiculaire de dioxaborolane et ses dérivés, en particulier 4,4,5,5-tetramethyle-1,3,2-dioxaborolane.

4. Procédé pour synthétiser des dérivés selon l'une quelconque des revendications 1-3, comprenant les étapes suivantes:
(i) synthèse d'un fluorène di-substitué en position 2 et 7;
(ii) ajoutant un imide d'halogène dans le mélange réactionnel contenant le fluorène di-substitué synthétisé dans l'étape (i), de préférence N-bromosuccinimide, et l'irradiation avec une lumière UV;
(iii) traitement du composé obtenu dans l'étape (ii) avec des métaux alcalins ou des métaux alcalino-terreux dans un environnement anhydre.

5. Procédé électrochimique pour synthétiser les anions radiculaires des dérivés selon l'une quelconque des revendications 1-3, **caractérisé en ce que** lesdites dérivés à être transformés en anions radiculaires sont ajoutés à une concentration comprise entre 0,01 M et 1 mM, de préférence entre 0,01 M et 0,5 mM, plus préférentiellement d'environ 1 mM, à un solvant aprotique anhydre contenant un électrolyte support, également anhydre, pour obtenir une concentration comprise du second entre 1 M et 0,01 M, de préférence de 0,2 M et 0,05 M, plus préférentiellement d'environ 0,1 M; où le mélange est ensuite placé dans une cellule électrolytique et une tension est appliquée entre les électrodes afin d'obtenir l'anion radiculaire requis.

6. Dispositifs électroniques et composants, choisis parmi: OLEDs, semi-conducteurs organiques, des transistors à effet de champ (OFET), redresseurs moléculaires, lasers; dispositifs photovoltaïques organiques; vannes de spin organiques, cellules solaires; matériaux électrochromes et thermochromiques pour des composants électroniques et des dispositifs à l'échelle moléculaire; composants et dispositifs pour les capteurs de gaz H₂; composants et dispositifs pour la production, la transmission et la détection des fréquences électromagnétiques également dans le domaine de l'infrarouge lointain; composants et dispositifs pour la spintronique, pour les capteurs chimiques et en général métamatériaux utiles pour les applications mentionnées ci-dessus, comprenant au moins l'un des dérivés et anions et cations radiculaires correspondants selon l'une quelconque des revendications 1-3, et leurs mélanges.

7. Utilisation des dérivés et des anions et cations radiculaires correspondants selon l'une quelconque des revendications 1-3, seul ou dans un mélange de ceux-ci, comme matériaux de transfert à charge négative (électrons) ou à charge positive (trous) ou comme matériaux de client dans des dispositifs électroniques.

8. Utilisation des dérivés et des anions et cations radiculaires correspondants selon la revendication 7, où lesdits dispositifs électroniques sont choisis parmi: des OLEDS, c.à.d. dispositifs pour les diodes électroluminescentes, en particulier émettant dans le visible, dans le bleu et le bleu-vert et émettant de la lumière blanche, en particulier pour les écrans de télévision ; des OFETS, c.à.d. transistors à effet de champ sur la base de composés organiques, des circuits intégrés sur la base de composés organiques, des cellules solaires sur la base de composés organiques; transistors d'émission de lumière sur la base de composés organiques ; cellules électrochimiques d'émission de lumière sur la base de composés organiques ; photorécepteurs organiques et laser diodes organiques; matériaux électrochromes, des vannes de spin organiques, des capteurs de gaz H₂.

9. Utilisation des dérivés et des anions et cations radiculaires correspondants selon l'une quelconque des revendications 1-3, seul ou en mélange, comme moyens actifs dans des dispositifs photovoltaïques et des dispositifs électroluminescents, y compris lasers ; et comme matériaux de transfert de charge dans des dispositifs électroluminescents; transistors, dispositifs de télécommunication, c.à.d. la production, transmission et détection de fréquences électromagnétiques et en général pour la production industrielle de métamatériaux utiles pour les applications mentionnées ci-dessus.
